# EUROPEAN PATENT APPLICATION

(11) **EP 3 653 716 A1**
(43) Date of publication of application: **20.05.2020**
(21) Application number: 18207121.7
(22) Date of filing: 19.11.2018
(51) Int. Cl.: C12N 15/869, A61K 39/12

(54) **REPLICATION-COMPETENT CONTROLLED ALPHA-HERPESVIRUS VIRUS VECTORS AND USES THEREFORE**

(71) Applicant: HSF Pharmaceuticals, 1814 La Tour-de-Peilz (CH)
(72) Inventor: VOELLMY, Richard, 1814 La Tour-de-Peilz (CH)
(74) Representative: Völlmy, Lukas

(57) **Abstract**

The present disclosure relates to a replication-competent controlled virus comprising a gene for a heterologous transactivator that is activated by a small-molecule regulator, the gene being functionally linked to a first heterologous promoter that is differentially active in cells in a tissue region of a mammalian subject to which the replication-competent controlled virus is intended to be administered when compared to cells in a nerve ganglion of the mammalian subject, and a second heterologous promoter that is responsive to the activated transactivator, the second heterologous promoter being functionally linked to a replication-essential gene of the replication-competent controlled virus.

## Description

### TECHNICAL FIELD

The present invention relates to certain replication-competent controlled alpha-herpesviruses and their utilization, including for immunization and cancer therapy.

### BACKGROUND OF THE INVENTION

Replication-competent controlled viruses are viruses whose replication is under deliberate control. They are capable of transiently replicating with near-wildtype efficiency upon activation but are essentially nonreplicating in the absence of activation. Typically, these viruses are derived from virulent DNA viruses by inserting into the viral genome an expressible gene for a small-molecule regulator-controlled heterologous transactivator and deliberately placing at least one replication-essential viral gene under the control of the latter transactivator. The present disclosure is concerned with viruses derived from virulent viruses of the alpha-herpesvirus subfamily, in particular mammalian HSV-1, HSV-2 and vaccinia viruses. While the primary targets of these viruses are mucoepithelial cells, their tropism is much broader. The viruses have a lytic phase during symptomatic infection as well as a latent phase where they lie dormant in sensory and cranial nerve ganglia.

Replication-competent controlled viruses have been described previously. For example, Chong et al. (2002) have described a complementing pair of adenoviruses, of which one virus expressed a gene for a rapamycin-controlled heterologous transactivator and the other contained an E1A gene that had been brought under control of a transactivator-responsive promoter (Chong et al. (2002) Mol Ther 5: 195-203). Other replication-competent controlled viruses and virus pairs were disclosed In U.S. Pat. Nos. 7,906,312 and 8,137,947, respectively. In the viruses of U.S. Pat. Nos. 7,906,312 (see also Bloom et al. (2015) J Virol 89: 10668-10679), a small-molecule regulator-controlled heterologous transactivator was expressed under the control of a heat shock promoter or a promoter cassette comprising a heat shock promoter and a transactivator-responsive promoter, and the promoters of one or more replication-essential genes were replaced by transactivator-responsive promoters.

Replication-competent controlled viruses may be used in oncolytic or other cancer therapy. They may also be employed as vaccines or vaccine vectors. As demonstrated in Bloom et al. (2018), the efficient replication of activated replication-competent controlled virus vectors substantially enhanced immune responses to the vectors or to heterologous antigens expressed by the vectors when compared to unactivated vectors or control viruses, respectively (Bloom et al. (2018) J Virol 92: e00616-18; see also U.S. Pat. Appl. No. 15/530,715).

### SUMMARY OF THE INVENTION

The present disclosure relates to a replication-competent controlled alpha-herpesvirus-derived virus whose replication is activated by a small-molecule regulator. Subsequent to administration to a tissue region of the body of a mammalian subject and in the presence of an effective concentration of the small-molecule regulator in the administration region, the replication-competent controlled virus replicates with an efficiency approaching that of the wildtype virus from which it had been derived. This replication is transient and ceases after the concentration in the administration region of the small-molecule regulator has fallen to an ineffective level (as a result of elimination by normal physiological processes). Over the ensuing weeks, virus is cleared from the body of the mammalian subject, except from sensory and, depending on the virus administration site, cranial nerve ganglia where it survives in a latent form. In the absence of the small-molecule regulator, reactivation from latency should not be capable of occurring. However, there is no reason for believing that reactivation cannot occur in the presence of an effective concentration of the small-molecule regulator in the body of the mammalian subject. Because reactivation is triggered by a physiological stress of some kind, it may be a rare and, typically, harmless event, especially if the region of administration has been chosen to minimize negative consequences of such reactivation. The small-molecule regulators of choice tend to be well known molecules that are readily available, have been tested in human toxicological studies and, ideally, are approved for human use. Such small-molecule regulators may not only be employed for the activation of replication-competent controlled viruses but may have other uses in medicine. Hence, although rare, reactivation of a latently present replication-competent controlled virus is a possibility. The replication-competent controlled viruses disclosed herein have been engineered to be resistant to reactivation even in the presence of their small-molecule regulators.

A replication-competent controlled virus of the present disclosure comprises inserted in its genome a gene for a heterologous transactivator that is capable of being activated by a small-molecule regulator. The latter transactivator gene is driven (or controlled) by a first heterologous promoter (or is functionally linked to the first heterologous promoter), which promoter is differentially active in cells in the tissue region of a mammalian subject to which tissue region the replication-competent controlled virus is intended to be administered when compared to (the activity of the latter promoter in) cells in a nerve ganglion, in particular the dorsal root ganglion, of the mammalian subject. The term "heterologous" when applied to a transactivator gene or a promoter means that the transactivator gene or the promoter is not naturally present in the genome of the wildtype virus from which the replication-competent controlled virus has been derived. The replication-competent controlled virus further comprises a second heterologous promoter that is responsive to the activated transactivator, i.e., that is activated by the transactivator in the presence of an effective concentration of a small-molecule regulator that is capable of activating the transactivator. The second heterologous promoter is functionally linked to a replication-essential viral gene and drives (or controls) the expression of the replication-essential viral gene. The viral promoter that drives the expression of the latter replication-essential viral gene in the wildtype virus from which the replication-competent controlled virus has been derived is substituted/replaced by the second heterologous promoter. In a replication-competent controlled virus of the present disclosure a second or further replication-essential viral gene may have been brought under the control of the second heterologous promoter by substitution of the respective viral promoter with the second heterologous promoter.

A first heterologous promoter may be identified in searches of pertinent databases of tissue-specific or selective expression of mammalian genes. One particularly useful database is the BIOGPS database (at www.biogps.org). The databases report transcript levels of individual genes in different tissues and organs, i.e., they disclose expression values that are averaged over the expression in all cells of a respective tissue or organ. The primary targets of alpha-herpesvirues of interest are mucoepithelial cells. Hence, a preferred region for administration of a replication-competent controlled virus of this disclosure may be a skin region in an extremity of a human subject. With this preference, the databases may be mined for genes that are active in the human epidermis but not or much less in human nerve ganglia, e.g., the dorsal root ganglia. Such a search may uncover the human keratin-1 gene as is discussed further below. The promoter of this gene may serve as a first heterologous promoter for a replication-competent controlled virus that is intended for administration to a skin region of a human subject.

Preferably, a first heterologous promoter will be a promoter that on average drives the transcriptional expression of the gene it is normally functionally linked to at least 10 times more effectively in cells in the tissue region of the body of a mammalian subject to which region the replication-competent controlled virus is intended to be administered than in cells of any other type of tissue (epithelial, connective, muscle, and nervous). With other words, the first heterologous promoter will be a promoter of a gene that, on average, is expressed at a level that is at least 10 times higher in the tissue region of the mammalian subject to which tissue region the replication-competent controlled virus is intended to be administered than in cells of any other type of tissue. In the example of the promoter of the human keratin-1 gene, the heratin-1 gene would be required to be at least 10 times more effectively expressed (>/=10 times higher average transcript levels) in the human epidermis than in human connective, muscle or nerve tissue.

Alternatively, a first heterologous promoter will be a promoter that on average drives the transcriptional expression of the gene it is normally functionally linked to at least 50 times more effectively in cells in the tissue region of the body of a mammalian subject to which region the replication-competent controlled virus is intended to be administered than in cells of nerve ganglia. Expressed differently, the first heterologous promoter will be a promoter of a gene that, on average, is expressed at a level that is at least 50 times higher in the tissue region of the mammalian subject to which tissue region the replication-competent controlled virus is intended to be administered than in cells of nerve ganglia. In the example of the promoter of the human keratin-1 gene, the heratin-1 gene would be required to be at least 50 times more effectively expressed (>/= 50 times higher average transcript levels) in the human epidermis than in cells of human nerve ganglia.

More preferably, a first heterologous promoter will be a promoter that on average drives the transcriptional expression of the gene it is normally functionally linked to at least 10 times more effectively in cells in the tissue region of the body of a mammalian subject to which region the replication-competent controlled virus is intended to be administered than in cells of any other type of tissue as well as at least 50 times more effectively than in cells of nerve ganglia. In the example of the promoter of the human keratin-1 gene, the human heratin-1 gene would be required to be at least 10 times more effectively expressed (>/= 10 times higher average transcript levels) in the human epidermis than in any other type of human tissue as well as at least 50 times more effectively (>/= 50 times higher average transcript levels) in the human epidermis than in cells of human nerve ganglia.

Most preferably, a first heterologous promoter will be a promoter that drives the transcriptional expression of the gene it is normally functionally linked in cells in the tissue region of the body of a mammalian subject to which region the replication-competent controlled virus is intended to be administered but has no detectable activity in cells of nerve ganglia. In other words, the first heterologous promoter is a promoter of a gene that is expressed in cells in the tissue region of the mammalian subject to which region the replication-competent controlled virus is intended to be administered but is not detectably expressed in cells of nerve ganglia.

A replication-competent controlled virus of the present disclosure can be derived from a (wildtype) herpes simplex virus 1 (HSV-1), herpes simplex virus 2 (HSV-2), or a varicella zoster virus (VZV).

In principle, the heterologous transactivator present in a replication-competent controlled virus of the present disclosure can be any transactivator that can be activated by a small-molecule regulator. Preferred is a transactivator that does not or only minimally affect host gene expression in a mammalian host and for which a small-molecule regulator is known that has no undue toxicity in the mammalian organism to which the replication-competent controlled virus is intended to be administered. As discussed further below, there are several transactivator/small-molecule regulator combinations that satisfy the latter requirements. Preferred is a transactivator that contains a ligand-binding domain from a progesterone receptor and is activated by a progesterone receptor antagonist (antiprogestin) or other molecule capable of interacting with the ligand-binding domain and of activating the transactivator. More preferred is a transactivator that contains a truncated ligand-binding domain from a progesterone receptor and is activated by a class of antiprogestins (including mifepristone and ulipristal) but not by progestins. The most preferred transactivator is GLP65.

In a replication-competent controlled virus of the present disclosure that has been derived from an HSV-1 or HSV-2, the viral gene ICP47 may be deleted or rendered nonfunctional. The product of this gene binds to transporters associated with antigen processing (TAP), interfering with the presentation of antigens to MHC class I molecules and, consequently, with immune recognition by cytotoxic T-lymphocytes.

A replication-competent controlled virus of the present disclosure can be engineered to carry in its genome an expressible gene from another pathogen, an expressible heterologous gene encoding an immune-modulatory polypeptide or an expressed heterologous gene encoding another polypeptide or any combination of one or more of such genes. The latter heterologous genes (i.e., genes not originally present in the wildtype virus from which the replication-competent controlled virus was derived) can be placed under the control of any suitable promoter, including a constitutively active viral or non-viral promoter, the first heterologous promoter or the second heterologous promoter.

Also encompassed by the present disclosure are vaccine compositions or compositions for cancer therapy or genetic therapy which compositions comprise an effective amount of a replication-competent controlled virus of the present disclosure and a pharmaceutically acceptable carrier or excipient. A composition comprising an effective amount of a small-molecule regulator that is capable of activating the heterologous transactivator expressed by the replication-competent controlled virus can be co-administered with any one of the latter compsitions or can be administered separately, including by a different route. For example, a vaccine composition can be administered topically to a region of the skin of a mammalian subject and a small-molecule regulator-comprising composition may be administered systemically (e.g., per os). Alternatively, a vaccine composition or a composition for cancer therapy or for genetic therapy comprising an effective amount of a replication-competent controlled virus and a pharmaceutically acceptable carrier or excipient may further comprise an effective amount of a small-molecule regulator that is capable of activating the transactivator comprised in the replication-competent controlled virus.

Also encompassed by the present disclosure are any uses of compositions comprising an effective amount of a replication-competent controlled virus of the present disclosure, optionally an effective amount of a small-molecule regulator that is capable of activating the transactivator expressed by the replication-competent controlled virus and a pharmaceutically acceptable carrier or excipient. These uses may relate to vaccination against the virus from which the replication-competent controlled virus was derived or, if a heterologous antigen of another pathogen is expressed from the replication-competent controlled virus, against that other pathogen. Alternatively, they may concern treatment, including oncolytic treatment, of a cancer (e.g., a melanoma) in a mammalian subject. Furthermore, they may relate to a genetic treatment of a condition or disease that responds to a protein expressed from an expressed heterologous gene carried by the replication-competent controlled virus.

Finally, the present disclosure also relates to derivatives of replication-competent controlled recombinant viruses whose genome comprises a gene for a small-molecule regulator-activated transactivator which gene is functionally linked to a nucleic acid sequence that acts as a heat shock promoter or to a nucleic acid sequence that acts as a heat shock promoter as well as a transactivator-responsive promoter, and a transactivator-responsive promoter that is functionally linked to a replication-essential viral gene. Replication-competent controlled viruses of this type were described in U.S. Pat. Nos. 7,906,312; U.S. Pat. Appl. No. 15/530,715; Bloom et al. (2015) and Bloom et al. (2018). Prototype viruses are also described under Example 1 of the present disclosure. These replication-competent controlled viruses can be engineered further to carry in their genomes an expressible gene from another pathogen, an expressible heterologous gene encoding an immune-modulatory polypeptide or an expressed heterologous gene encoding another polypeptide or any combination of one or more of such genes. The latter heterologous genes (i.e., genes not originally present in the wildtype virus from which the replication-competent controlled virus was derived) can be placed under the control of any suitable promoter, including a constitutively active viral or non-viral promoter or a transactivator-responsive promoter. The recombinant viruses can be derived from a virus selected from an HSV-1, an HSV-2, a varicella zoster virus, a cytomegalovirus and a roseola virus. The transactivator in the exemplified recombinants is an antiprogestin-activated transactivator. Analogous recombinants can be prepared using different small-molecule regulator-activated transactivators as described hereinafter. A replication-competent controlled virus of the latter type may enter latent phase in cells of nerve ganglia in a subject to which the replication-competent controlled virus has been administered. Reactivation from latency may conceivably occur when the subject is undergoing a treatment that involves administration of the small-molecule regulator that co-controls the replication of the virus. This possibility of reactivation can be minimized by placing a further replication-essential gene (in addition to the gene that is under transactivator control) under the control of the afore-mentioned first heterologous promoter that is active in the tissue (e.g., epithelial) region of a subject to which region the replication-competent controlled virus is administered but not active or less active in nerve ganglia. The construction of such derivative recombinants is described under Example 8. Also encompassed are compositions comprising such derivative recombinants and uses thereof.

### DETAILED DESCRIPTION

Unless otherwise defined below or elsewhere in the present specification, all terms shall have their ordinary meaning in the relevant art.

"Replication of virus" or "virus/viral replication" are understood to mean multiplication of viral particles. Replication is often measured by determination of numbers of infectious virus, e.g., plaque-forming units of virus (pfu). However, replication can also be assessed by biochemical methods such as methods that determine amounts of viral DNA, e.g., by a realtime PCR procedure, levels of viral gene expression, e.g., by RT-PCR of gene transcripts, etc. However, it is understood that marginal increases in levels of viral DNA or viral gene transcripts or protein products may not translate in corresponding marginal increases in virus replication due to threshold effects.

A "small-molecule regulator" is understood to be a low molecular weight ligand of a transactivator used in connection with this invention. The small-molecule regulator is capable of activating the transactivator. The small-molecule regulator is typically, but not necessarily, smaller than about 1000 Dalton (1 kDa).

The terms "transactivator" or "heterologous transactivator" are used herein to refer to a non-viral and, typically, engineered transcription factor that when activated by the appropriate small-molecule regulator can positively affect transcription of a gene controlled by a transactivator-responsive promoter.

"Activated" when used in connection with a transactivated gene means that the rate of expression of the gene is measurably greater after activation than before activation. When used in connection with a transactivator, "active" or "activated" refers to a transactivation-competent form of the transactivator. The transactivator is rendered transactivation-competent by the binding of the appropriate small-molecule regulator.

Herein, a virus, whose genome includes a foreign (heterologous) non-viral or viral gene, is either referred to as a "virus" or a "viral vector".

A "replication-competent controlled virus" is a recombinant virus whose replicative ability is under the control of a gene switch that can be deliberately activated.

A "recombinant virus" or "recombinant" refers to a virus that has been altered by an experimenter.

A "replication-essential gene" or a "gene required for efficient replication" is arbitrarily defined herein as a viral gene whose loss of function diminishes replication efficiency by a factor of ten or greater. Replication efficiency can be estimated, e.g., in a (single-step) growth experiment. For many viruses it is well known which genes are replication-essential genes. For herpesviruses see, e.g., Nishigawa (1996) Herpesvirus genes: molecular basis of viral replication and pathogenicitiy. Nagoya L Med Sci 59: 107-19.

An "effective amount of a replication-competent controlled virus" is an amount of virus that upon single or repeated administration to a subject followed by activation confers a therapeutic effect on the treated subject, at a reasonable benefit/risk ratio applicable to any medical treatment. When used in the context of prophylaxis or prevention, an "effective amount" of a replication-competent controlled virus of the disclosure is meant to be an amount which, when administered (and thereafter activated) once or multiple times over the course of a prophylactic or prevention (e.g., vaccination) regime, confers a desired prophylactic effect on the treated subject. In general, what is an "effective amount" will also vary depending on route of administration, as well as the possibility of co-usage with other agents. It will be understood, however, that the total or fractional usage of compositions of the present disclosure comprising a replication-competent controlled virus will be decided by the attending physician within the scope of sound medical judgment. The specific effective dose level for any particular patient may be adjusted based on a variety of factors including the disorder being treated and the severity of the disorder; the activity of the replication-competent controlled virus employed; the specific composition employed; the age, body weight, general health, sex and diet of the patient; the time of administration, route of administration, and rate of elimination of the specific replication-competent controlled virus employed; the duration of the treatment; drugs used in combination or contemporaneously with the replication-competent controlled virus employed; and like factors well known in the medical arts. The latter factors will be considered in the context of therapeutic applications of replication-competent controlled viruses as well as in the context of prophylactic or preventative applications.

An "effective amount of a small-molecule regulator" is an amount that when administered to a subject by a desired route is capable of activating a small-molecule regulator-activated virus (i.e., a replication-competent controlled virus of the present disclosure) with which the subject concurrently is, has been or will be inoculated to undergo at least one round of replication in the administration site region.

A "subject" or a "mammalian subject" is a mammalian animal or a human person.

The present invention relates to replication-competent controlled viruses that, upon activation, replicate with efficiencies that approach those of the respective wild type viruses. A wild type HSV-1, HSV-2 or varicella zoster virus (VZV) is genetically altered by placing at least one selected replication-essential gene under the control of a gene switch that has a broad dynamic range, i.e., that essentially functions as an on/off switch. The gene switch comprises a transactivator that is activated by a small-molecule regulator but is essentially inactive in the absence of this small-molecule regulator. Hence, the wild type virus is engineered to contain an expressible gene for such a transactivator in a location in the viral genome in which an insertion of such a gene does not interfere with virus function, in particular with replication efficiency. The UL43/44 intergenic region is such a location. The promoter of a selected replication-essential gene is substituted by a transactivator-responsive promoter that is activated by the small-molecule regulator-activated transactivator but is essentially inactive when not contacted by the activated transactivator. Wildtype HSV-1, HSV-2 or VZV are known to preferentially target mucoepithelial cells and to establish latency in cells of nerve ganglia, e.g., in cells of the dorsal root ganglia. The replication-competent controlled viruses of the present disclosure are engineered to replicate efficiently in mucoepithelial cells (and derived cells), e.g., epidermal cells of the skin, but not (or only minimally) in cells of nerve ganglia. This is achieved by the use of an appropriate tissue- or cell type-specific or restricted (heterologous) promoter for controlling the expression of the inserted transactivator gene. It is noted that the tissue specificity of regulation of replication of the replication-competent controlled virus can be further enhanced by replacement of the endogenous promoter of a further replication-essential gene with the latter tissue- or cell type-specific or restricted promoter (as exemplified by recombinant HSV-GS3E; see under Examples).

A tissue- or cell type-specific or restricted promoter that is appropriate for the intended use of a replication-competent controlled virus can be identified from mining available databases and other scientific literature.

For a replication-competent controlled virus that is to be used as a vaccine or a vaccine vector administered to a region of the skin of a human subject, a promoter that is highly active in all epidermal layers of the human skin but not (or only minimally) in cells of nerve ganglia may be selected for driving transactivator expression. A particular promoter that may be employed is the human KRT1 promoter (Edqvist et al. (2015) Expression of human skin-specific genes defined by transcriptomics and antibody-based profiling. J Histochem Cytochem 63: 129-41). Inspection of the BIOGPS database (at www.biogps.org) reveals that this promoter is essentially only expressed in skin (and other epithelia as discussed below). No expression is evident in nerve ganglia. Another useful promoter is that of the human KRT10 gene. Transcript levels are far higher in the skin than in any other tissue/organ. Essentially no expression occurs in nerve ganglia. For a vaccine to be administered to the skin of a murine subject, a suitable promoter may be that of the mouse KRT77 gene. The latter gene is highly active in the mouse epidermis but not in any other adult tissue. No expression occurs in the dorsal root ganglia. Other useful promoters may by the mouse KRT1 and KRT10 promoters for which no activity can be demonstrated in nerve ganglia. That they are also active in the stomach may not significantly detract from their usefulness in vaccine applications. The sequences of the latter promoters can be found in the Eukaryotic Promoter Database and elsewhere.

For a human vaccine directed against genital herpes or other sexually transmitted diseases that is to be administered to the vaginal mucosal epithelium with the intention of inducing resident immunity, reference is made to Borgdorff et al. (2016) Mucosal Immunol 9: 621-33. The latter publication reports that the KRT1, KRT4, KRT5, KRT6A, KRT10 and KRT13 genes are abundantly expressed in the epithelial layer of the vagina. Preferred promoters for driving the transactivator gene (of a replication-competent controlled virus) in such an application are those of the KRT1, KRT4 and KRT13 genes that are most selectively active and appear to have essentially no activity in nerve ganglia.

For an oncolytic therapy of human melanoma, in particular primary melanoma, a suitable promoter for driving the transactivator gene may be that of the human MLANA gene or, possibly, that of the human TYR gene. Weinstein et al. (2014) J Clin Aesthet Dermatol 7: 13-24. Both promoters typically have elevated activity in skin melanomas (Human Protein Atlas) but only minimal activity in nerve ganglia.

Promoters for controlling expression of transactivator genes comprised in replication-competent controlled viruses of the present disclosure, including the promoters specifically disclosed above, are selected because they are active in the intended target cells but essentially inactive in cells of nerve ganglia to preclude the possibility of reactivation of the viruses from latency. It is conceivable that, even when using such selective promoters, an unacceptable level of replication in neural cells is detected (which is a level that enables detectable reactivation from latency in a subject). If this occurs, it is likely that the promoter concerned is also excessively active in the target cells, i.e., highly efficient (wildtype-like) replication could be had at a considerably lower level of promoter activity. Hence, to reduce viral replication in neural cells, it may be indicated to reduce the level of transactivator expression enabled by the promoter. Various engineering approaches to achieve this goal are known in the art. For example, protein-destabilizing elements could be introduced into the transactivator, e.g., near the carboxy terminus of the protein. Well known sequence elements of this type are the so-called PEST sequences that are thought to function as proteolytic signals. Rechsteiner & Rogers (1996) Trends Biochem Sci 21: 267-71. These sequences contain regions enriched in proline (P), glutamate (E), serine (S) and threonine (T). PEST sequences are hydrophilic stretches of at least 12 amino acids in length, with the entire region flanked by lysine (K), arginine (R) or histidine (H), but not interrupted by positively charged residues. RNA-destabilizing elements, AU-rich elements (ARE), may be added to the 3'UTR sequence of the transactivator gene. Such elements were described in Zubiaga et al. (1995) Mol Cell Biol 15: 2219-30. See also Matoulkova et al. (2012) RNA Biol 9: 563-76. RNA- and protein-destabilizing elements have also been used in combination to dramatically reduce protein levels (Voon et al. (2005) Nucleic Acids Res 33 (3): e27). Other approaches are aimed at reducing translation efficiency. The introduction of highly stable secondary structure (hairpins) near the 5' end of the gene (close to the 5' methyl G cap) can dramatically reduce translation efficiency as shown by Babendure et al. (2006) RNA 12: 851-61. Hence, such secondary structure elements could be introduced into the transactivator gene to reduce transactivator expression.

A small-molecule regulator should satisfy a number of criteria. Most important will be that the substance is safe; adverse effects should occur at most at an extremely low rate and should be generally of a mild nature. Ideally, the chosen small-molecule regulator will belong to a chemical group that is not used in human therapy. However, before any substance not otherwise developed for human therapy could be used as a small-molecule regulator in a medical application of a replication-competent controlled virus vector, it would have to undergo extensive preclinical and clinical testing. It may be more efficacious to select a known and well-characterized drug substance that is not otherwise administered to the specific population targeted for treatment or immunization by a replication-competent controlled virus. Alternatively, a known drug substance may be selected that will not need to be administered to subjects within at least the first several weeks after treatment or immunization. Thus, a potential low-level risk is further reduced by the avoidance of administration of the drug substance during the period during which the replication-competent controlled virus is systemically present. Sporadic use of the drug substance under medical supervision will ensure that any significant inadvertent replication of replication-competent controlled virus would be rapidly diagnosed and antiviral measures could be taken without delay. In the examples described herein, the small-molecule regulator is a progesterone receptor (PR) antagonist or antiprogestin, e.g., mifepristone or ulipristal. Mifepristone and ulipristal fulfill the latter requirement of not typically needing to be administered shortly after virus administration. Mifepristone and ulipristal have excellent safety records.

An effective concentration of a small-molecule regulator in the inoculation site region is a concentration that enables replication (at least one round) of a replication-competent controlled virus in infected cells of that region. What an effective concentration is depends on the affinity of the small-molecule regulator for its target transactivator. How such effective concentration is achieved and for how long it is maintained also depends on the pharmacokinetics of the particular small-molecule regulator, which in turn depends on the route of administration of the small-molecule regulator, the metabolism and route of elimination of the small-molecule regulator, the subject to which the small-molecule regulator is administered, i.e., the type of subject (human or other mammal), its age, condition, weight, etc. It further depends on the type of composition administered, i.e., whether the composition permits an immediate release or a slow release of the regulator. For a number of well-characterized small-molecule regulator-transactivator systems, effective concentrations in certain experimental subjects have been estimated and are available from the literature. This applies to systems based on progesterone receptor, ecdysone receptors, estrogen receptors, and tetracycline repressor as well as to dimerizer systems, i.e., transactivators activated by rapamycin or analogs (including non-immunosuppressive analogs), or FK506 or analogs. For example, an effective concentration of mifepristone in rats can be reached by i.p. (intraperitoneal) administration of 5 µg mifepristone per kg body weight (5 µg/kg). Amounts would have to be approximately doubled (to about 10 µg/kg), if the small-molecule regulator is administered orally. Wang, Y. et al. (1994) Proc Natl Acad Sci USA 91: 8180-84. Amounts of a small-molecule regulator that, upon administration by the chosen route, result in an effective concentration are referred to as effective amounts of the small-molecule regulator in question. How an effective amount of a small-molecule regulator that results in an effective concentration can be determined is well within the skills of an artisan.

A replication-competent controlled virus of the present disclosure (a small-molecule regulator-activated virus) and an appropriate small-molecule regulator can be co-administered in a single composition. Replication-competent controlled virus and small-molecule regulator can also be administered in separate compositions. Topical coadministration of replication-competent controlled virus and small-molecule regulator appears advantageous for several reasons, including minimization of potential secondary effects of the small-molecule regulator, further reduction of the already remote possibility that virus may replicate systemically during the immunization period, and minimization of the environmental impact of elimination of small-molecule regulator. Notwithstanding these advantages, the small-molecule regulator may be given by a systemic route, e.g., orally, which may be preferred if a formulation of the drug substance of choice is already available that has been tested for a particular route of administration. The relative timing of inoculation with replication-competent controlled virus and administration of an effective amount of the appropriate small-molecule regulator is derivative of the operational requirements of transactivator control. Regarding administration of the small-molecule regulator, there typically will be a certain degree of flexibility because it will be possible to maintain an effective concentration systemically or specifically in the inoculation site region for one to several days. Consequently, small-molecule regulator can be administered prior to, at the time of or subsequent to virus administration, the only requirement being that the regulator be present in an effective concentration in the inoculation site region for the time needed for the transactivator to fulfill its role in transiently enabling viral replication. Typically, this time will correspond to that required for the completion of a desired (low) number of replication cycles. Minimally, it will correspond to the time required for the completion of one round of induced virus replication. Typically, a round of virus replication is completed within about one day.

Inoculation can be by any suitable route. The body region (inoculation site region) to which replication-competent controlled virus is administered may typically be a cutaneous or subcutaneous region located anywhere on the trunk or the extremities of a subject. Preferably, administration of a composition of the invention comprising a replication-competent controlled virus may be to a cutaneous or subcutaneous region located on an upper extremity of the subject. Administration may also be to the lungs or airways, a mucous membrane in an orifice of a subject or any other tissue region in which the virus is capable of replicating.

The replication-competent controlled viruses disclosed herein are controlled by an antiprogestin-activated gene switch. They incorporate mifepristone (and ulipristal)-dependent chimeric transactivator GLP65 (or glp65). This transactivator comprises a DNA-binding domain from yeast transcription factor GAL4, a truncated ligand-binding domain from a human progesterone receptor and a transactivation domain from the human RelA protein (p65). Burcin et al. (1999) Proc Natl Acad Sci USA 96: 355-60; Ye et al. (2002) Meth Enzymol 346: 551-61. Other exemplary small-molecule regulator-activated transactivators than can be incorporated in replication-competent controlled viruses include tetracycline/doxycycline-regulated tet-on repressors (Gossen & Bujard (1992) Proc Natl Acad Sci USA 89: 5547-51; Gossen et al. (1996) Science 268: 1766-69), and transactivators containing a ligand-binding domain of an insect ecdysone receptor. No et al. (1996) Proc Natl Acad Sci USA 93: 3346-51. A stringently ligand-dependent transactivator of this type is the RheoSwitch transactivator developed by Palli and colleagues. Palli et al. (2003) Eur J Biochem 270: 1308-15; Kumar et al. (2004) J Biol Chem 279: 27211-18. The RheoSwitch transactivator can be activated by ecdysteroids such as ponasterone A or muristerone A, or by synthetic diacylhydrazines such as RSL-1 (also known as RH-5849, first synthesized by Rohm and Haas Company). Dhadialla et al. (1998) Annu Rev Entomol 43: 545-69. Other small molecule-regulated transactivators may be used, provided that they can be employed to control the activity of a target gene without also causing widespread deregulation of genes in cells of the intended hosts (subjects) and provided further that the associated small-molecule regulators have acceptably low toxicity for the hosts at their effective concentrations.

Another concern has been whether pre-existing immunity to a virus will preclude its use as a vaccine or oncolytic vector. This issue not only relates to viruses such as adenoviruses and herpesviruses that are endemic but also to viruses that not normally infect humans but are used repeatedly as vectors. There may have been more serious concerns regarding the effects of pre-existing immunity to adenovirus (type 5) than to any other vector. Draper & Heeney (2010) Nat Rev Microbiol 8: 62-73. A recent study demonstrated that pre-existing immunity does not interfere with the generation of memory CD8 T cells upon vaccination with a heterologous antigen-expressing modified Ad5 vector, providing a basis for an efficient recall response and protection against subsequent challenge. Steffensen et al. (2012) PLoS ONE 7: e34884. Furthermore, the transgene product-specific response could be boosted by re-vaccination. The issue of pre-existing immunity to herpesviruses has also been examined in multiple studies. Brockman & Knipe (2002) J Virol 76: 3678-87; Chahlavi et al. (1999) Gene Ther 6: 1751-58; Delman et al. (2000) Hum Gene Ther 11: 2465-72; Hocknell et al. (2002) J Virol 76: 5565-80; Lambright et al. (2000) Mol Ther 2: 387-93; Herrlinger et al. (1998) Gene Ther 5: 809-19; Lauterbach et al. (2005 J Gen Virol 86: 2401-10; Watanabe et al. (2007) Virology 357: 186-98. A majority of these studies reported little effect or only relatively minor effects on immune responses to herpesvirus-delivered heterologous antigens or on anti-tumor efficacy of oncolytic herpesviruses. Brockman & Knipe (2002); Chahlavi et al. (1999); Delman et al. (2000); Hocknell et al. (2002); Lambright et al. (2000); Watanabe, D (2007). Two studies were identified that reported substantial reductions of immune responses. Herrlinger (1998); Lauterbach et al. (2005). However, it appears that the results of these studies may not be generalized because compromised models were employed. One of the studies employed a tumor model that was only barely infectable with the mutant HSV strain used. Herrlinger (1998). The other study employed a chimeric mouse immune model in combination with a severely crippled HSV strain (ICP4⁻, ICP22⁻, ICP27⁻, vhs-) as the test vaccine. Lauterbach et al. (2005). All studies agreed that vaccine uses of herpesviruses are possible even in the presence of pre-existing immunity. It may be added that pre-existing immunity, e.g., to herpesviruses, may not be a general issue for childhood preventative or therapeutic interventions.

Viruses have evolved a multitude of mechanisms for evading immune detection and avoiding destruction. Tortorella et al. (2000) Annu Rev Immunol 18: 861-926. Elimination or weakening of some of these mechanisms could further enhance the potency of a replication-competent controlled virus. For example, HSV-1 and HSV-2 express protein ICP47. This protein binds to the cytoplasmic surfaces of both TAP1 and TAP2, the components of the transporter associated with antigen processing TAP. Advani & Roizman (2005) In: Modulation of Host Gene Expression and Innate Immunity by Viruses (ed. P. Palese), pp. 141-61, Springer Verlag. ICP47 specifically interferes with MHC class I loading by binding to the antigen-binding site of TAP, competitively inhibiting antigenic peptide binding. Virus-infected human cells are expected to be impaired in the presentation of antigenic peptides in the MHC class I context and, consequently, to be resistant to killing by CD8+ CTL. Deletion or disablement of the gene that encodes ICP47 ought to significantly increase the potency of a replication-competent controlled virus (both as an oncolytic agent and as a vaccine).

The potency of a replication-competent controlled virus may also be enhanced by including in the viral genome an expressible gene for a cytokine or other component of the immune system. A vaccination study in mice in which replication-defective herpesvirus recombinants expressing various cytokines were compared demonstrated that virus-expressed IL-4 and IL-2 had adjuvant effects. Osiorio & Ghiasi (2003) J Virol 77: 5774-83. Further afield, modulation of dendritic cell function by GM-CSF was shown to enhance protective immunity induced by BCG and to overcome non-responsiveness to a hepatitis B vaccine. Nambiar et al. (2009) Eur J Immunol 40: 153-61; Chou et al. (2010) J Immunol 185: 5468-75.

Expanding upon the basic definition given on p.7 as it relates to vaccine uses, an effective amount of a replication-competent controlled virus of the invention is an amount that upon administration to a subject and induced replication therein results in a detectably enhanced functional immunity of the subject (that is typically superior to the immunity induced by a replication-defective comparison virus). This enhanced functional immunity may manifest itself as enhanced resistance to infection or re-infection with a circulating (wild type) virus or may relate to enhanced suppression/elimination of a current infection. Hence, it may also manifest itself by a reduced disease severity, disease duration or mortality subsequent to infection with said wild type virus. Alternatively, or in addition, in the case of an immunizing virus expressing a foreign (heterologous) antigen, immunity can relate to preventive or therapeutic immunity against pathogens expressing and/or displaying the latter foreign antigen. It is noted that a number of factors will influence what constitutes an effective amount of a replication-competent controlled virus, including to some extent the site and route of administration of the virus to a subject as well as the precise activation regimen utilized. Effective amounts of a replication-competent controlled virus will be determined in dose-finding experiments. Generally, for vaccine uses, an effective amount of a replication-competent controlled virus of the invention will be from about 10² to about 10⁸ plaque-forming units (pfu) of virus. More preferably, an effective amount will be from about 10³ to about 10⁷ pfu of virus, and even more preferably from about 10³ to about 10⁶ pfu of virus. Larger amounts may be indicated, in particular for oncolytic therapies.

A composition of the invention will comprise an effective amount of a replication-competent controlled virus and, if a small-molecule regulator is also administered as part of the composition, an effective amount of the small-molecule regulator. It further comprises, typically, a pharmaceutically acceptable carrier or excipient. Although it may be administered in the form of a fine powder, e.g., a lyophilizate, under certain circumstances (see, e.g., U.S. Pat. Appl. Publ. No 20080035143; Chen et al. (2017) J Control Release 255: 36-44), a composition of the invention typically is an aqueous composition comprising a virus of the invention and, as the case may be, a small-molecule regulator. It may be administered parenterally to a subject as an aqueous solution or, in the case of administration to a mucosal membrane, possibly as an aerosol thereof. See, e.g., U.S. Pat. No. 5,952,220. The compositions of the present invention will typically include a buffer component. The compositions will have a pH that is compatible with the intended use and is typically between about 6 and about 8. A variety of conventional buffers may be employed such as phosphate, citrate, histidine, Tris, Bis-Tris, bicarbonate and the like and mixtures thereof. The concentration of buffer generally ranges from about 0.01 to about 0.25% w/v (weight/volume).

The compositions of the invention comprising a replication-competent controlled virus can further include, for example, preservatives, virus stabilizers, tonicity agents and/or viscosity-increasing substances. As mentioned before, they may also include an appropriate small-molecule regulator, or a formulation comprising such small-molecule regulator.

Preservatives used in parenteral products include phenol, benzyl alcohol, methyl paraben/propylparaben and phenoxyethanol. Phenoxyethanol is the most widely used preservative found in vaccines. Preservatives are generally used in concentrations ranging from about 0.002 to about 1 % w/v. Meyer (2007) J Pharm Sci 96: 3155-67. Preservatives may be present in compositions comprising a replication-competent controlled virus at concentrations at which they do not or only minimally interfere with the replicative efficiency of the virus.

Osmolarity can be adjusted with tonicity agents to a value that is compatible with the intended use of the compositions. For example, the osmolarity may be adjusted to approximately the osmotic pressure of normal physiological fluids, which is approximately equivalent to about 0.9 % w/v of sodium chloride in water. Examples of suitable tonicity adjusting agents include, without limitation, chloride salts of sodium, potassium, calcium and magnesium, dextrose, glycerol, propylene glycol, mannitol, sorbitol and the like, and mixtures thereof. Preferably, the tonicity agent(s) will be employed in an amount to provide a final osmotic value of 150 to 450 mOsm/kg, more preferably between about 220 to about 350 mOsm/kg and most preferably between about 270 to about 310 mOsm/kg.

If indicated, the compositions of the invention can further include one or more viscosity-modifying agents such as cellulose polymers, including hydroxypropylmethyl cellulose, hydroxyethyl cellulose, ethylhydroxyethyl cellulose, hydroxypropyl cellulose, methyl cellulose, carboxymethyl cellulose, glycerol, carbomers, polyvinyl alcohol, polyvinyl pyrrolidone, alginates, carrageenans, guar, karaya, agarose, locust bean, tragacanth and xanthan gums. Such viscosity modifying components are typically employed in an amount effective to provide the desired degree of thickening. Viscosity-modifying agents may be present in compositions comprising a replication-competent controlled virus at concentrations at which they do not or only minimally interfere with the replicative efficiency of the virus.

If the composition also contains a small-molecule regulator, an effective amount of such small-molecule regulator can be included in the composition in the form of a powder, solution, emulsion or particle. As also provided before, an effective amount of a small-molecule regulator to be co-delivered with an effective amount of a replication-competent controlled virus will be an amount that yields an effective concentration of small-molecule regulator in the inoculation site region, which effective concentration enables at least one round of replication of the replication-competent controlled virus in infected cells of that region. To maintain a small-molecule regulator at an effective concentration for a more extended period, it may be included in the form of a slow-release formulation (see also below).

Methods for amplifying viruses are well known in the laboratory art. Industrial scale-up has also been achieved. For herpesviruses, see Hunter (1999) J Virol 73: 6319-26; Rampling et al. (2000) Gene Ther 7: 859-866; Mundle et al. (2013) PLoS ONE 8(2): e57224. Various methods for purifying viruses have been disclosed. See, e.g., Mundle et al. (2013) and references cited therein; Wolf & Reichl (2011) Expert Rev Vaccines 10: 1451-75.

While a small-molecule regulator can be co-administered with a replication-competent controlled virus in a single composition, a composition comprising a replication-competent controlled virus and a composition comprising a small-molecule regulator can also be administered separately. The latter composition will comprise an effective amount of a small-molecule regulator formulated together with one or more pharmaceutically acceptable carriers or excipients.

A composition comprising a small-molecule regulator may be administered orally, parenterally, by inhalation spray, topically, rectally, nasally, buccally, vaginally or via an implanted reservoir, preferably by oral administration, administration by injection or deposition at the site of virus inoculation. The compositions may contain any conventional non-toxic, pharmaceutically acceptable carriers, adjuvants or vehicles. In some cases, the pH of the formulation may be adjusted with pharmaceutically acceptable acids, bases or buffers to enhance the stability of the formulated small-molecule regulator or its delivery form. The term parenteral as used herein includes subcutaneous, intracutaneous (epidermis and/or dermis), intravenous, intramuscular, intraarticular, intraarterial, intrasynovial, intrasternal, intrathecal, intralesional and intracranial injection or infusion techniques.

Liquid dosage forms of a small-molecule regulator for oral administration include pharmaceutically acceptable emulsions, microemulsions, solutions, suspensions, syrups and elixirs. In addition, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include, e.g., wetting agents, emulsifying and suspending agents, sweetening, flavoring, and perfuming agents.

Injectable preparations, for example sterile injectable aqueous or oleaginous suspensions, may be formulated according to the known art using suitable dispersing or wetting agents and suspending agents. The sterile injectable preparation may also be a sterile injectable solution, suspension or emulsion in a nontoxic, parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

In order to prolong the effect of a small-molecule regulator, it may be desirable to slow the absorption of the compound from, e.g., subcutaneous, intracutaneous or intramuscular injection. This may be accomplished by the use of a liquid suspension of crystalline or amorphous material with poor water solubility. The rate of absorption of the small-molecule regulator then depends upon its rate of dissolution, which, in turn, may depend upon crystal size and crystalline form. Alternatively, delayed absorption of a parenterally administered small-molecule regulator is accomplished by dissolving or suspending the compound in an oil vehicle. Injectable depot forms are made by forming microcapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration can be suppositories which can be prepared by mixing the small-molecule regulator with suitable non-irritating excipients or carriers such as cocoa butter, polyethylene glycol or a suppository wax which are solid at ambient temperature but liquid at body temperature and therefore melt in the rectum or vaginal cavity and release the small-molecule regulator.

Solid dosage forms for oral administration include capsules, tablets, pills, powders, and granules. In such solid dosage forms, the small-molecule regulator is mixed with at least one inert, pharmaceutically acceptable excipient or carrier such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the small-molecule regulator only, or preferentially, in a certain part of the intestinal tract, optionally, in a delayed manner. Examples of embedding compositions that can be used include polymeric substances and waxes.

Dosage forms for topical, intradermal or transdermal administration of a small-molecule regulator include ointments, pastes, creams, lotions, gels, powders, solutions, sprays, inhalants or patches. The small-molecule regulator is admixed under sterile conditions with a pharmaceutically acceptable carrier and any preservatives or buffers as may be required.

The ointments, pastes, creams and gels may contain, in addition to a small-molecule regulator, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the small-molecule regulator, excipients such as lactose, talc, silicic acid, aluminum hydroxide, calcium silicates and polyamide powder, or mixtures of these substances. Sprays can additionally contain customary propellants such as hydrofluorocarbons.

Transdermal patches have the added advantage of providing controlled delivery of a compound. Such dosage forms can be made by dissolving or dispensing the compound in the proper medium. Absorption enhancers can also be used to increase the flux of the compound into or across the skin.

For pulmonary delivery, a composition comprising an effective amount of a small-molecule regulator of the invention is formulated and administered to the subject in solid or liquid particulate form by direct administration e.g., inhalation into the respiratory system. Solid or liquid particulate forms of the small-molecule regulator prepared for practicing the present invention include particles of respirable size: that is, particles of a size sufficiently small to pass through the mouth and larynx upon inhalation and into the bronchi and alveoli of the lungs. Delivery of aerosolized therapeutics, particularly aerosolized antibiotics, is known in the art (see, for example U.S. Pat. Nos. 5,767,068 and 5,508,269, and WO 98/43650). A discussion of pulmonary delivery of antibiotics is also found in U.S. Pat. No. 6,014,969.

What an effective amount of a small-molecule regulator is will depend on the activity of the particular small-molecule regulator employed, the route of administration, time of administration, the stability and rate of excretion of the particular small-molecule regulator as well as the nature of the specific composition administered. It may also depend on the age, body weight, general health, sex and diet of the subject, other drugs used in combination or contemporaneously with the specific small-molecule regulator employed and like factors well known in the medical arts.

Ultimately, what is an effective amount of a small-molecule regulator has to be determined in dose-finding experiments, in which viral replication is assessed experimentally in the inoculation site region. Once an effective amount has been determined in animal experiments, it may be possible to estimate a human effective amount. "Guidance for Industry. Estimating the maximum safe starting dose for initial clinical trials for therapeutics in adult healthy volunteers", U.S. FDA, Center for Drug Evaluation and Research, July 2005, Pharmacology and Toxicology. For example, as estimated from rat data, an effective human amount of orally administered mifepristone (for enabling at least one cycle of virus replication) will be between about 1 and about 100 µg/kg body weight.

Recitation of ranges of values herein are merely intended to serve as a shorthand method of referring individually to each separate value falling within the range, unless otherwise indicated herein, and each separate value is incorporated into the specification as if it were individually recited herein.

The description herein of any aspect or embodiment of the invention using terms such as reference to an element or elements is intended to provide support for a similar aspect or embodiment of the invention that "consists of'," "consists essentially of" or "substantially comprises" that particular element or elements, unless otherwise stated or clearly contradicted by context (e. g. , a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

The present invention, thus generally described, will be understood more readily by reference to the following examples, which are provided by way of illustration and are not intended to be limiting of the present invention.

### EXAMPLES

### Example 1: Construction of replication-competent controlled virus HSV-GS3 that is coactivated by heat and antiprogestin

All vectors were constructed using wild type HSV-1 strain 17*syn*+ as the backbone. This strain is fully virulent, is well characterized, and the complete genomic sequence is available. The generation of the viral recombinants was performed by homologous recombination of engineered plasmids along with purified virion DNA typically into rabbit skin cells (RS) by the calcium phosphate precipitation method as previously described. Bloom, D. C. 1998. HSV Vectors for Gene Therapy. Methods Mol. Med. 10: 369-386. All plasmids used to engineer the insertions of the transactivators or the GAL4-responsive promoters for recombination into the HSV-1 genome were cloned from HSV-1 strain 17*syn*+*.*

HSV-GS3 had been constructed as described in detail in Bloom et al. (2015) Replication-competent controlled herpes simplex virus. J Virol 89: 10668-79, and Voellmy et al. (2017) Development of recombinant HSV-based vaccine vectors. Methods Mol Biol 1581: 55-78. Briefly, an Hsp70/GAL4-GLP65 transactivator (TA) cassette was isolated from plasmid Hsp70/GAL4-GLP65 (Vilaboa et al. (2005) Novel gene switches for targeted and timed expression of proteins of interest. Mol Ther: 12: 290-8) and subcloned into plasmid IN994 (Bloom et al. 2015, Voellmy et al. 2017; see also below). One µg of the resulting transfer plasmid construct pIN:TA1 was co-transfected with 2 µg of purified HSV-1 (17+) virion DNA into rabbit skin (RS) cells by calcium phosphate precipitation. A recombinant virus was isolated that was designated HSV-17GS43. This recombinant contained an Hsp70/GAL4-GLP65 TA cassette inserted in the intragenic region between UL43 and UL44. (As disclosed before, GLP65 contains a GAL4 DNA binding domain, a truncated progesterone receptor ligand-binding domain and transcription activation domains from p65.) Plasmid IN994 contains, separated by a multiple cloning site region, HSV-1 DNA (17+) segments from nt 95,441 to 96,090 and nt 96,092 to 96,538, and recombines with HSV-1 at the intergenic UL43/44 region.

Transfer plasmid BS-KS:GAL4-ICP4 was constructed by subcloning a GAL4-responsive promoter retrieved from plasmid Gene/v5-HisA (Invitrogen Corp.) into plasmid BS-KS:ICP4Δpromoter. The latter plasmid contained ICP4 recombination arms but lacked the ICP4 promoter sequence. Recombinant HSV-GS1 was isolated from RS cells co-transfected with plasmid BS-KS:GAL4-ICP4 and HSV-17GS43 virion DNA. HSV-GS1 contains the aforementioned TA cassette and ICP4 genes that are functionally linked to GAL4-responsive promoters. Transfer plasmid BS-KS:GAL4-ICP8 was constructed by inserting a GAL4-responsive promoter in between the HSV-1 ICP8 recombination arms in plasmid BS-KS:ICP8Δpromoter (that lacks the ICP8 promoter). Recombinant HSV-GS3 was isolated from RS cells co-transfected with plasmid BS-KS:GAL4-ICP8 and HSV-GS1 virion DNA. HSV-GS3 contains the aforementioned TA cassette and has both ICP4 and ICP8 genes functionally linked to GAL4-responsive promoters.

### Example 2: Construction of replication-competent controlled virus HSV-GS3B

Recombinant HSV-GS3B contains a mouse KRT77 promoter-driven GLP65 transactivator (TA) gene inserted into the intergenic region between UL43 and UL44. In addition, the ICP4 promoter is replaced with a GAL4-responsive promoter (GAL4-binding site-containing minimal promoter) in both copies of the short repeats, and the ICP8 promoter is replaced with a GAL4-responsive promoter. The promoter sequence of the mouse keratin-77 (KRT77) gene was retrieved from the Eukaryotic Promoter Database (EPD; https://epd.vital-it.ch/index.php). A DNA segment beginning about 1'000 bp upstream from the transcription start site and ending 50-85 bp downstream from the transcription start site is PCR-amplified from C57BL/6 mouse DNA and subcloned in a plasmid such as pUC19 (New England Biolabs). The correctness of the subcloned DNA is verified by restriction digestion and nucleotide sequence analysis. The complete GLP65 gene is PCR-amplified from plasmid pSwitch (Invitrogen Life Technologies; map provided in the Invitrogen manual for the GeneSwitch System available at https://www.thermofisher.com; nucleotide sequence at http://www.yrgene.com/documents/ vector/pswitch_seq.txt) using suitable primers. The GLP65 segment and the KRT77 promoter/RNA leader segment retrieved from its plasmid vector are co-inserted by three-piece ligation into plasmid IN994. The resulting transfer plasmid pIN:TA2 contains the KRT77 promoter/RNA leader region that is linked at its RNA leader end to the 5'nontranslated sequence end of the GLP65 gene as verified by restriction and nucleotide sequence analysis.

One µg of pIN:TA2, one µg of pSwitch and 2 µg of purified HSV-GS3 virion DNA are co-transfected into rabbit skin (RS) cells by calcium phosphate precipitation in the presence of mifepristone (10 nM). The resulting pool of viruses is screened for recombinants by picking plaques, amplifying these plaques on 96 well plates of RS cells (transfected 24 h prior to infection with pSwitch) in the presence of mifepristone, and dot-blot hybridization with a ³²P-labeled DNA probe prepared by labeling a KRT77 promoter/RNA leader fragment by random-hexamer priming. A positive well is re-plaqued and re-probed 5 times and verified to contain the KRT77-TA cassette by PCR and sequence analysis. This recombinant is designated HSV-GS3B. The above-described procedure can be used, mutatis mutandis, for the preparation of recombinants in which the GLP65 TA gene is controlled by a different mammalian promoter.

### Example 3: Analysis of differential regulated replication of HSV-GS3B in epidermal and neural cells

Stocks of HSV-GS3B can be prepared by routine procedures in pSwitch-transfected RS cells or in COCA epidermal cells (see below) in the presence of mifepristone.

Replication of recombinant HSV-GS3B and wildtype viral strain 17*syn*+ is compared in the mouse skin epithelial cell line COCA (Segrelles et al. (2011) BMC Dermatology 11: 9) and mouse neural cell line Neuro-2a, in the presence or absence of mifepristone. Coca cells (obtained from Sigma-Aldrich) are grown in CnT-07 medium (CELLnTEC, Bern, Switzerland). Neuro-2a cells (obtained from the American Tissue Culture Collection ATCC) are grown in Eagle's Minimum Essential Medium plus 10% FBS. The ability of HSV-1 to infect and replicate in Neuro-2a cells has been documented previously (Parker et al. (2000) Proc Natl Acad Sci USA 97: 2208-13).

Confluent monolayers of target cells are infected with virus (HSV-GS3B or 17syn+) at a multiplicity of infection (MOI) of 3. Virus is allowed to adsorb for 1 h at 37°C, and then the inoculum is removed, and the cells are overlayed with medium. Mifepristone treatment (10 nM) is initiated at the time of the initial infection. The dishes are then incubated at 37°C. At 0, 4, 12 and 24 h post infection, two dishes are removed, and the cells are scraped into medium to harvest and subjected to 2 freeze-thaw cycles. Infectious virus is then determined by titrating the lysate of each dish in triplicate on 24-well plates of confluent E5 cells (ICP4-complementing cell line; DeLuca and Schaffer (1987) Nucleic Acids Res 15: 4491-4511) transfected 24 h prior to infection with expression plasmid plCP8 using Lipofectamine 2000 (Life Technologies). Plaques are visualized after 2 days by staining with crystal violet. Alternatively, plaques are visualized 2 days after infection using an antibody plaque assay, essentially as previously described (Bewig and Schmidt (2000) Biotechniques 28: 870-873). Briefly, medium is removed, and the monolayers fixed with cold 100% methanol for 20 min at 20°C. The monolayers are then washed with phosphate-buffered saline (PBS) and, subsequent to addition of 100 µl of a 1/1000 dilution of a polyclonal anti-HSV antiserum (rabbit anti-HSV HRP conjugate, DAKO labs), are incubated for 1 h a room temperature. The antiserum is aspirated, the monolayers are rinsed twice with PBS, and the plaques are visualized by adding 200 µl of Immunopure DAB substrate (Pierce Chemicals), which is removed after approximately 10 min by rinsing with PBS.

To construct expression plasmid plCP8, a 3777 bp fragment containing the entire ICP8-coding sequence and the native promoter of the ICP8 gene was PCR-amplified from HSV-1 17syn+ virion DNA and subcloned into pBS-KS:ΔSacI. The plasmid insert was verified by sequence analysis. To obtain pBS-KS:ΔSacI, the SacI site was deleted from the polylinker of plasmid vector pBluescript-KS+, by digesting the plasmid with Sacl.

Representative results of growth experiments of the kind described above (but testing different replication-competent controlled viruses in different cell types) were disclosed, e.g., in Bloom et al. (2015) (Fig.2 & 3). In the instantly described growth experiments, recombinant HSV-GS3B is expected replicate with similar efficiency as wildtype virus 17syn+ in the epidermal cells in the presence of mifepristone. Essentially no replication of HSV-GS3B should be apparent in the absence of mifepristone. In the neural cells, whereas 17syn+ should replicate well, replication of HSV-GS3B should not be observed, either in the presence or the absence of mifepristone.

### Example 4: Reactivation from latency

Herpesviruses are known to latently infect sensory nerve cells in which they can reactivate under adverse circumstances, e.g., during a high fever. In HSV-GS3B (and its derivatives) expression of two replication-essential genes is controlled by an antiprogestin-activated transactivator that is expressed from a gene driven by a promoter known to be virtually inactive in neural cells. Hence, reactivation of HSV-GS3B in sensory neurons should not occur in a subject, even if the subject is experiencing a high fever or other severe stress and is concurrently administered an antiprogestin. This assumption is tested in a well-established mouse model of heat-induced reactivation (Sawtell & Thompson (1992) J Virol 66: 2150-2156). In this model, all mice that had been inoculated on the lightly abraded rear footpads with HSV-1 wildtype virus strain 17syn+ were found to contain latent virus in their ganglia at 30 days after inoculation. No spontaneous reactivation was observed. Heat treatment reactivated virus in the dorsal root ganglia (DRG) in about 80% of latently infected animals.

To demonstrate that HSV-GS3B does not reactivate, 4 groups (n = 10) of 4- to 6-week-old Swiss Webster mice are inoculated on the abraded rear footpads with 250 pfu of wildtype HSV-1 strain 17syn+ (2 groups) or 50,000 pfu of HSV-GS3B (2 groups). Thirty days later, all animals will receive mifepristone (0.5 mg/kg) intraperitoneally, and one of the 17syn+ groups and one of the HSV-GS3B groups are subjected to heating in a 43°C water bath as described in Sawtell & Thompson (1992). Individual animals or small groups are slowly lowered into the water. Body temperature increase is monitored by means of a rectally inserted thermocouple. Heat treatments are for 10 min (with body temperature reaching 43°C after about 7 min). The mice are then allowed to recover at 37°C for 15 min. Twenty-four hours later, all animals are euthanized. Dorsal root ganglia (DRG) are recovered, homogenized and subjected to a freeze-thaw cycle to lyse cells. After clarification, the extracts are used to infect confluent cultures of plCP8-transfected E5 cells. The cultures are then monitored daily for cytopathic effects (reactivation).

### Example 5: Construction of replication-competent controlled virus HSV-GS3C

Recombinant HSV-GS3C contains a mouse KRT77 promoter-driven GLP65 transactivator (TA) gene inserted into the intergenic region between UL43 and UL44. In addition, the ICP4 promoter is replaced with a GAL4-responsive promoter (GAL4-binding site-containing minimal promoter) in both copies of the short repeats, and the ICP8 promoter is replaced with a GAL4-responsive promoter. Furthermore, the US12 gene is mutated to render its protein product (ICP47) nonfunctional. ICP47 amino acid residue K31 is changed to G31, and R32 to G32. Neumann et al. (1997) J Mol Biol 272: 484-92; Galocha et al. (1997) J Exp Med 185: 1565-72. A 500 bp ICP47 coding sequence-containing fragment is PCR-amplified from virion DNA of strain 17*syn*+. The fragment is PCR-amplified as two pieces (a "left-hand" and a "right-hand" piece), using two primer pairs. The mutations are introduced through the 5' PCR primer for the right-hand fragment. The resulting amplified left-hand and mutated right-hand fragments are subcloned into vector pBS, and the sequence of a selected subclone is confirmed by sequence analysis. The subclone containing the 500 bp fragment with the desired mutations in ICP47 codons 31 and 32 is termed pBS:mut-ICP47.

One µg of pBS:mut-ICP47 is co-transfected with 10 µg of purified HSV-GS3B virion DNA into pSwitch-transfected RS cells by calcium phosphate precipitation. Subsequent to the addition of mifepristone to the medium, the transfected cells are incubated at 37°C. Plaques are picked and amplified on 96 well plates of plCP8-transfected E5 cells. The plates are incubated at 37°C. After the wells show 90 - 100% CPE, the plates are dot-blotted and the dot-blot membrane hybridized with a ³²P-labeled oligonucleotide probe to the mutated ICP47 region. A positive well is re-plaqued and re-probed several times and verified by sequence analysis to contain the expected mutated ICP47 gene sequence. This recombinant is designated HSV-GS3C.

### Example 6: Construction of replication-competent controlled virus HSV-GS3D

Recombinant HSV-GS3D contains a mouse KRT77 promoter-driven GLP65 transactivator (TA) gene inserted into the intergenic region between UL43 and UL44. In addition, the ICP4 promoter is replaced with a GAL4-responsive promoter (GAL4-binding site-containing minimal promoter) in both copies of the short repeats, and the ICP8 promoter is replaced with a GAL4-responsive promoter. Furthermore, the recombinant contains an insertion between the UL37 and UL38 genes of a gene cassette expressing the EIV Prague/56 hemagglutinin (HA) gene driven by the CMV IE promoter. A recombination plasmid was constructed by the following sequential steps. First, a 814 bp fragment containing the region spanning the HSV-1 UL37/UL38 intergenic region from nt 83,603-84,417 from plasmid NK470 was subcloned into pBS that had had the MCS removed (digestion with Kpnl/Sacl) to yield pBS:UL37/38. A cassette containing a synthetic CMV IE promoter flanked by the pBS-SK+ MCS was ligated into pBS:UL37/38 digested with BspE1/Aflll, which enzymes cut between the UL37 and UL38 genes, to yield the plasmid pIN:UL37/38. The EIV Prague/56 HA gene was PCR-cloned from cDNA prepared from EIV Prague/56. Briefly, RNA was prepared by Trizol extraction of a stock of EIV Prague 56 and was reverse-transcribed using Omni-Script Reverse Transcriptase (Qiagen) according to the manufacturer's instructions. The cDNA was cloned into pBS, and the clone containing the HA gene (pBS-EIVPrague56/HA) was confirmed by sequence analysis. The Prague/56 HA gene was retrieved from this plasmid and inserted behind the CMV promoter in the plasmid pIN:UL37/38 to yield plasmid pIN:37/38-Prague56/HA.

To produce recombinant HSV-GS3D, pSwitch-transfected RS cells are co-transfected with plasmid pIN:37/38-Prague56/HA and purified HSV-GS3B virion DNA. Subsequent to the addition of mifepristone to the medium, the co-transfected cells are incubated at 37°C. Picking and amplification of plaques, screening and plaque purification are performed essentially as described for HSV-GS3B, except that dot-blot hybridization is with a ³²P-labeled DNA probe prepared by labeling an EIVPrague56/HA fragment by random-hexamer priming. The resulting plaque-purified HSV-GS3D is verified by Southern blot as well as by PCR and DNA sequence analysis of the recombination junctions.

### Example 7: Construction of replication-competent controlled virus HSV-GS3E

Recombinant HSV-GS3E contains a mouse KRT77 promoter-driven GLP65 transactivator (TA) gene inserted into the intergenic region between UL43 and UL44. In addition, the ICP4 promoter is replaced with a GAL4-responsive promoter (GAL4-binding site-containing minimal promoter) in both copies of the short repeats, and the ICP8 promoter is replaced with a GAL4-responsive promoter. Furthermore, the UL38/VP19c gene is controlled by a mouse KRT77 promoter.

To place the UL38/VP19c gene under regulation of a mouse KRT77 promoter, plasmid pBS-KS:KRT77-UL38 is constructed. This plasmid contains a mKRT77 promoter/RNA leader sequence (see under Example 2 for details) inserted in the multicloning site region between the HSV-1 UL38 recombination arms of plasmid pBS-KS:UL38Δpromoter. Plasmid pBS-KS:UL38Δpromoter was constructed by deletion of the region from -1 to -47 of the UL38 promoter, i.e., by synthesizing two PCR fragments (one 437 bp and the other 550 bp long) on either side of the deletion and cloning these into pBS KS+.To produce recombinant HSV-GS3E, pSwitch-transfected RS cells are co-transfected with plasmid pBS-KS:KRT77-UL38 and purified HSV-GS3B virion DNA. Subsequent to the addition of mifepristone (10 nM), the co-transfected cells are incubated at 37°C. Picking and amplification of plaques, screening and plaque purification is performed essentially as described for HSV-GS3B, except that (negative) dot-blot hybridization is with a ³²P-labeled synthetic DNA probe containing part of the sequence of the deleted UL38 promoter segment. The resulting plaque-purified HSV-GS3E is verified by Southern blot as well as by PCR and DNA sequence analysis of the recombination junctions.

### Example 8: Construction of HSV-GS1A and HSV-GS3A

Recombinant HSV-GS1 contains, inserted in the intergenic region between UL43 and UL44, a GLP65 transactivator (TA) gene that is under the (dual) control of an HSP70 and a GAL4-responsive promoter. In addition, the ICP4 promoter is replaced with a GAL4-responsive promoter (GAL4-binding site-containing minimal promoter) in both copies of the short repeats. In HSV-GS3, derived from HSV-GS1, the ICP8 promoter is also replaced with a GAL4-responsive promoter. In HSV-GS1A and HSV-GS3A, derived from HSV-GS1 and HSV-GS3 respectively, the UL38/VP19c gene is controlled by a mouse KRT77 promoter.

To produce recombinants HSV-GS1A or HSV-GS3A, pSwitch-transfected RS cells are co-transfected with plasmid pBS-KS:KRT77-UL38 (described under the preceding example) and purified HSV-GS1 virion DNA (for HSVGS1A) or HSV-GS3 virion DNA (for HSVGS3A). Subsequent to the addition of mifepristone (10 nM), co-transfected cells are incubated at 37°C. Picking and amplification of plaques, screening and plaque purification is performed essentially as described for HSV-GS3B. The resulting plaque-purified HSV-GS1A and HSV-GS3A are verified by Southern blot as well as by PCR and DNA sequence analysis of the recombination junctions.

Generally known molecular biology and biochemistry methods are/were used. Molecular biology methods are described, e.g., in "Current protocols in molecular biology", Ausubel, F.M. et al., eds., John Wiley and Sons, Inc. ISBN: 978-0-471-50338-5.

## Claims

1. A replication-competent controlled virus comprising
(a) a gene for a heterologous transactivator that is activated by a small-molecule regulator, the gene being functionally linked to a first heterologous promoter that is differentially active in cells in a tissue region of a mammalian subject to which tissue region the replication-competent controlled virus is intended to be administered when compared to cells in a nerve ganglion of the mammalian subject, and
(b) a second heterologous promoter that is responsive to the activated transactivator, the second heterologous promoter being functionally linked to a replication-essential gene of the replication-competent controlled virus.

2. The replication-competent controlled virus of claim 1, wherein the first heterologous promoter is a promoter of a gene that, on average, is expressed at a level that is at least 10 times higher in the tissue region of the mammalian subject to which tissue region the replication-competent controlled virus is intended to be administered than in cells of any other type of tissue or at least 50 times higher than in cells of nerve ganglia.

3. The replication-competent controlled virus of claim 1, wherein the first heterologous promoter is a promoter of a gene that, on average, is expressed at a level that is at least 10 times higher in the tissue region of the mammalian subject to which tissue region the replication-competent controlled virus is intended to be administered than in cells of any other type of tissue and at least 50 times higher than in cells of nerve ganglia.

4. The replication-competent controlled virus of claim 1, wherein the first heterologous promoter is a promoter of a gene that is expressed in cells in the tissue region of the mammalian subject to which region the replication-competent controlled virus is intended to be administered but is not detectably expressed in cells of nerve ganglia.

5. The replication-competent controlled virus of any of claims 1-4, wherein the replication-competent controlled virus is derived from a virus selected from the group consisting of an HSV-1, an HSV-2 and a varicella zoster virus.

6. The replication-competent controlled virus of any of claims 1-5, wherein the small-molecule regulator-activated transactivator contains a ligand-binding domain from a progesterone receptor and is activated by an antiprogestin or other molecule capable of interacting with the ligand-binding domain and of activating the transactivator.

7. The replication-competent controlled virus of any of claims 1-4 and 6, wherein the replication-competent controlled virus is derived from an HSV-1 or HSV-2 and is lacking a functional ICP47 gene.

8. The replication-competent controlled virus of any of claims 1-7 further comprising at least one of an expressed gene from another pathogen, an expressed heterologous gene encoding an immune-modulatory polypeptide and an expressed heterologous gene encoding another polypeptide.

9. A vaccine composition comprising an effective amount of a replication-competent controlled virus of any of claims 1-8 and a pharmaceutically acceptable carrier or excipient.

10. A composition for cancer therapy comprising an effective amount of a replication-competent controlled virus of any of claims 1-8 and a pharmaceutically acceptable carrier or excipient.

11. A composition for genetic therapy comprising an effective amount of a replication-competent controlled virus of any of claims 1-7, which virus carries an expressible therapeutic gene, and a pharmaceutically acceptable carrier or excipient, optionally wherein the composition further comprises an effective amount of a small-molecule regulator that is capable of activating the transactivator comprised in the replication-competent controlled virus.

12. The composition of any one of claims 9, 10 or 11, further comprising an effective amount of a small-molecule regulator that is capable of activating the transactivator comprised in the replication-competent controlled virus.

13. A replication-competent controlled virus of any of claims 1-7, for use in preventative or therapeutic vaccination against a disease caused by the virus from which replication-competent controlled virus was derived.

14. A replication-competent controlled virus of any of claims 1-7:
(a) further comprising an expressed gene from another pathogen, wherein the virus is for use in preventative or therapeutic vaccination against diseases caused by said another pathogen; or
(b) further comprising an expressed therapeutic gene for therapy, wherein the virus is for use as a medicament for treatment of a patient in need of the product of the therapeutic gene.

15. A replication-competent controlled virus of any of claims 1-8, for use in an oncolytic treatment of a cancer in a mammalian subject.
